# NEW EUROPEAN PATENT SPECIFICATION

(11) **EP 0 637 998 B2**
(45) Date of publication and mention of the opposition decision: **12.04.2006**
(45) Mention of the grant of the patent: 31.07.1996
(21) Application number: 93910891.6
(22) Date of filing: 29.04.1993
(51) Int. Cl.: B01L 3/00, C12M 3/08, G01N 15/10

(54) **FLUID HANDLING IN MICROFABRICATED ANALYTICAL DEVICES**
FLUESSIGKEITSBEHANDLUNG IN MIKROFABRIZIERTEN ANALYTISCHEN VORRICHTUNGEN
MANIPULATION DE FLUIDES DANS DES DISPOSITIFS D'ANALYSE MICRO-USINES

(30) Priority: 01.05.1992 US 877536; 01.05.1992 US 877661; 01.05.1992 US 877662; 01.05.1992 US 877701; 01.05.1992 US 877702
(43) Date of publication of application: 15.02.1995
(73) Proprietor: TRUSTEES OF THE UNIVERSITY OF PENNSYLVANIA, Philadelphia, PA 19104 (US)
(72) Inventor: WILDING, Peter, Paoli, PA 19301 (US); KRICKA, Larry, J., Berwyn, PA 19312 (US); ZEMEL, Jay, N., Jenkintown, PA 19046 (US)
(74) Representative: Armitage, Ian Michael
(86) International application number: PCT/US1993/004018
(87) International publication number: WO 1993/022055

(56) References cited:
- DE-A- 4 028 771
- US-A- 4 350 768
- US-A- 4 676 274
- 1988 IEEE INDUSTRY APPLICATIONS SOCIETY ANNUAL MEETING vol. 2, October 1988, ATLANTA (US) pages 1735 - 1740 WASHIZU ET AL. 'handling of biological cells using fluid integrated circuit' cited in the application
- 1987 IEEE INDUSTRY APPLICATIONS SOCIET ANNUAL MEETING vol. 2, October 1987, ATLANTA (US) pages 1549 - 1553 MASUDA ET AL. 'novel method of cell fusion in field constriction area in fluid integrated circuit' cited in the application
- Kricka et al., "Liquid Transport in Micron and Submicron Channels", SPIE, 1167:159-168 (1989)

## Description

### Background of the Invention

This invention relates generally to apparatus for conducting analyses. More particularly, the invention relates to the design and construction of small, typically single-use, modules capable of analyzing a fluid sample.

In recent decades the art has developed a very large number of protocols, test kits, and cartridges for conducting analyses on biological samples for various diagnostic and monitoring purposes. Immunoassays, agglutination assays, and analyses based on polymerase chain reaction, various ligand-receptor interactions, and differential migration of species in a complex sample all have been used to determine the presence or concentration of various biological compounds or contaminants, or the presence of particular cell types.

Recently, small, disposable devices have been developed for handling biological samples and for conducting certain clinical tests. Shoji et al. reported the use of a miniature blood gas analyzer fabricated on a silicon wafer. Shoji et al., Sensors and Actuators, 15:101-107 (1988). Sato et al. reported a cell fusion technique using micromechanical silicon devices. Sato et al., Sensors and Actuators, A21-A23:948-953 (1990). Ciba Corning Diagnostics Corp. (USA) has manufactured a microprocessor-controlled laser photometer for detecting blood clotting.

Micromachining technology originated in the microelectronics industry. Angell et al., Scientific American, 248:44-55 (1983). Micromachining technology has enabled the manufacture of microengineered devices having structural elements with minimal dimensions ranging from tens of microns (the dimensions of biological cells) to nanometers (the dimensions of some biological macromolecules). This scale is referred to herein as "mesoscale". Most experiments involving mesoscale structures have involved studies of micromechanics, i.e., mechanical motion and flow properties. The potential capability of mesoscale structures has not been exploited fully in the life sciences.

Brunette (Exper. Cell Res., 167:203-217 (1986) and 164:11-26 (1986)) studied the behavior of fibroblasts and epithelial cells in grooves in silicon, titanium-coated polymers and the like. McCartney et al. (Cancer Res., 41:3046-3051 (1981)) examined the behavior of tumor cells in grooved plastic substrates. LaCelle (Blood Cells, 12:179-189 (1986)) studied leukocyte and erythrocyte flow in microcapillaries to gain insight into microcirculation. Hung and Weissman reported a study of fluid dynamics in micromachined channels, but did not produce data associated with an analytic device. Hung et al., Med. and Biol. Engineering, 9:237-245 (1971); and Weissman et al., Am. Inst. Chem. Eng. J., 17:25-30 (1971). Columbus et al. utilized a sandwich composed of two orthogonally orientated v-grooved embossed sheets in the control of capillary flow of biological fluids to discrete ion-selective electrodes in an experimental multichannel test device. Columbus et al., Clin. Chem., 33:1531-1537 (1987). Masuda et al. and Washizu et al. have reported the use of a fluid flow chamber for the manipulation of cells (e.g. cell fusion). Masuda et al., Proceedings IEEE/IAS Meeting, pp. 1549-1553 (1987); and Washizu et al., Proceedings IEEE/IAS Meeting pp. 1735-1740 (1988). The art has not fully explored the potential of using mesoscale devices for the analyses of biological fluids and detection of microorganisms.

The current analytical techniques utilized for the detection of microorganisms are rarely automated, usually require incubation in a suitable medium to increase the number of organisms, and invariably employ visual and/or chemical methods to identify the strain or sub-species. The inherent delay in such methods frequently necessitates medical intervention prior to definitive identification of the nature of an infection. In industrial, public health or clinical environments, such delays may have serious consequences. There is a need for convenient systems for the rapid detection of microorganisms.

An object of the invention is to provide analytical systems with optimal reaction environments that can analyze microvolumes of sample, detect substances present in very low concentrations, and produce analytical results rapidly. Another object is to provide easily mass produced, disposable, small (e.g., less than 1 cc in volume) devices having mesoscale functional elements capable of rapid, automated analyses in a range of biological and other applications. It is a further object of the invention to provide a family of such devices that individually can be used to implement a range of rapid clinical tests, e.g., tests for bacterial contamination, virus infection, sperm motility, blood parameters, contaminants in food, water, or body fluids, and the like.

### Summary of the Invention

The invention provides devices for the analysis of a fluid sample. The device comprises a solid substrate, typically on the order of a few millimeters thick and approximately 0.2 to 2.0 centimeters square, micro-fabricated to define a sample inlet port and a mesoscale flow system. The mesoscale flow system includes a sample flow channel, extending from the inlet port, and a fluid handling region, in fluid communication with the flow channel. The term "mesoscale" is used herein to define chambers and flow passages having cross-sectional dimensions on the order of 0.1 µm to 500 µm. The mesoscale flow channels and fluid handling regions have preferred depths on the order of 0.1 µm to 100 µm, typically 2 - 50 µm. The channels have preferred widths on the order of 2.0 to 500 µm, more preferably 3 - 100 µm. For many applications, channels of 5 - 50 µm widths will be useful. Chambers in the substrates often will have larger dimensions, e.g., a few millimeters.

In one embodiment, the device may be utilized to analyze a cell containing fluid sample, and the fluid handling region may comprise a cell handling region. The device may further include means for inducing flow of cells in the sample through the mesoscale flow system. The cell handling region may comprise a cell lysis means. The flow inducing means may be utilized to force a cell sample through the cell lysis means to rupture the cells. Means may also be provided in the device for detecting the presence of an intracellular molecular component of a cell in the cell sample. The cell lysis means may comprise, e.g., sharp-edged pieces of silicon trapped within the cell handling region, or cell membrane piercing protrusions extending from a wall of the cell handling region of the mesoscale flow system. Alternatively, a region of reduced cross-sectional area may comprise the cell lysis means. The flow system may further comprise a microfabricated filter for, e.g., filtering cellular debris from the sample, prior to analysis for the presence of an intracellular analyte.

The cell handling region may also comprise a cell capture region comprising binding sites capable of reversibly binding a cell surface molecule to enable the selective isolation of a cell population from a cell sample. Means may also be provided downstream of the cell capture region for determining the presence of a cell or cell surface molecule in the sample. In another embodiment, the cell handling region may comprise an inert barrier, such as posts extending from a wall of the region, to enable the sorting of cells by size. The posts also may comprise, e.g., a barrier to the flow of a sperm sample, to enable the assessment of sperm motility.

Generally, as disclosed herein, the solid substrate comprises a chip containing the mesoscale flow system. The mesoscale flow system may be designed and fabricated from silicon and other solid substrates using established micromachining methods. The mesoscale flow systems in the devices may be constructed by microfabricating flow channels and one or more fluid handling regions into the surface of the substrate, and then adhering a cover, e.g., a transparent glass cover, over the surface. The devices typically are designed on a scale suitable to analyze microvolumes (<10 µL) of sample, introduced into the flow system through an inlet port defined, e.g., by a hole communicating with the flow system through the substrate or the cover. The volume of the mesoscale flow system typically will be <5 µm, and the volume of individual channels, chambers, or other functional elements are often less than 1 µm, e.g., in the nL or pL range. Cells or other components present in very low concentrations (e.g., nanogram quantities) in microvolumes of a sample fluid can be rapidly analyzed (e.g., <10 minutes).

The chips typically will be used with an appliance which contains a nesting site for holding the chip, and which mates one or more input ports on the chip with one or more flow lines in the appliance. After a fluid sample, e.g., a cell-containing fluid sample, suspected to contain a particular cell type, or molecular component, is applied to the inlet port of the substrate, the chip is placed in the appliance, and a pump, e.g., in the appliance, is actuated to force the sample through the flow system. Alternatively, a sample may be injected into the chip by the appliance. The sample also may enter the flow system by capillary action.

In one embodiment, the fluid handling chamber of the device may include a mesoscale detection region, downstream from the fluid handling region, for detecting the presence of an analyte in the fluid sample such as a cellular, intracellular, or other fluid sample component. The detection region may be constructed in accordance with USSN 07/877,702, filed May 1, 1992 (corresponding to PCT WO 93/22053, published November 11, 1993). The appliance may be designed to receive electronic or spectrophotometric signals in the detection region, to indicate the presence of the preselected component in the cell sample. The presence of a cellular, intracellular or other analyte in the detection region may also be detected optically, e.g., through a transparent or translucent window, such as a transparent cover, over the detection region, or through a translucent section of the substrate itself. The appliance may include sensing equipment such as a spectrophotometer, capable of detecting the presence of a preselected analyte in the detection region. In one embodiment, the detection region may comprise binding moieties, capable of binding to the analyte to be detected, thereby to enhance and facilitate detection. The detection region also may comprise a fractal region, i.e., a region of serially bifurcating flow channels, sensitive to changes in flow properties of a fluid sample, as is disclosed in USSN 07/877,701, filed May 1, 1992 (corresponding to PCT WO 93/22054, published November 11, 1993). The device also may be fabricated with at least three inlet ports, in fluid communication with the flow system, provided with valves, e.g., in an appliance used in combination with the device, for closing and opening the ports to enable the control of fluid flow through the mesoscale flow system.

The mesoscale devices can be adapted to perform a wide range of biological tests. Some of the features and benefits of the devices are summarized in Table 1. A device may include two or more separated flow systems, e.g., fed by a common inlet port, with different cell handling chambers in each of the systems to enable two or more analyses to be conducted simultaneously. The devices can be utilized to implement a range of rapid tests, e.g., to detect the presence of a cellular or intracellular component of a fluid sample. The devices may be utilized to detect, e.g., a pathogenic bacteria or virus, or for cell sorting. The invention provides devices for a wide range of possible analyses. Assays may be completed rapidly, and at the conclusion of the assay the chip can be discarded, which advantageously prevents contamination between samples, entombs potentially hazardous materials, and provides inexpensive, micro-sample analyses.

**TABLE 1**

| Feature | Benefit |
|---|---|
| Flexibility | No limits to the number of chip designs or applications available. |
| Reproducible | Allows reliable, standardized, mass production of chips. |
| Low Cost Production | Allows competitive pricing with existing systems. Disposable nature for single-use processes. |
| Small Size | No bulky instrumentation required. Lends itself to portable units and systems designed for use in non-conventional lab environments. Minimal storage and shipping costs. |
| Microscale | Minimal sample and reagent volumes required. Reduces reagent costs, especially for more expensive, specialized test procedures. Allows simplified instrumentation schemes. |
| Sterility | Chips can be sterilized for use in microbiological assays and other procedures requiring dean environments. |
| Sealed System | Minimizes biohazards. Ensures process integrity. |
| Multiple Circuit Capabilities | Can perform multiple processes or analyses on a single chip. Allows panel assays. Multiple Expands capabilities for assay and Detector process monitoring to virtually any Capabilities system. Allows broad range of applications. Reuseable Chips Reduces per process cost to the user for certain applications. |

### Brief Description of the Drawings

FIGURE 1 is a magnified plan view of a device according to the invention that includes a solid substrate 14, on which are machined entry ports 16, mesoscale flow channel 20, cell lysis chamber 22, and fractal region 40, with a transparent cover 12 adhered to the surface of the substrate.
FIGURE 2 is a longitudinal cross sectional view of the device shown in Figure 1.
FIGURE 3 is a perspective view of the device of Figure 1.
FIGURE 4 is a schematic illustration of analytical device 10 nested within appliance 50, which is used to support the device 10 and to regulate and detect the pressure of sample fluids in device 10.
FIGURE 5 is a cross sectional perspective view of a fluid handling region 22 on the inert substrate 14 with cell or debris filtering protrusions 26 extending from the wall of the flow channel.
FIGURE 6 is a cross sectional view of a fluid handling region 22 on the inert substrate 14 with cell piercing protrusions 24 extending from the wall of the channel.
FIGURE 7 is a schematic top view of an analytical device 10 fabricated with a series of mesoscale chambers suitable for implementing a variety of functions including cell sorting, cell lysing and PCR analysis.
FIGURES 8 through 10 illustrate different embodiments of a filter microfabricated in a mesoscale flow channel 20.
FIGURES 11 is schematic perspective view of an apparatus 60 used in combination with device 10 for viewing the contents of device 10.
FIGURE 12 is a schematic cross sectional view of the apparatus 60 of Figure 11.

Like reference characters in the respective drawn figures indicate corresponding parts.

### Detailed Description

The invention provides apparatus for the analysis of a fluid sample. The device comprises a solid substrate, microfabricated to define a sample inlet port and a mesoscale flow system. The mesoscale flow system comprises a sample flow channel extending from the inlet port, and a fluid handling region in fluid communication with the flow channel. In one embodiment, the devices may be utilized to analyse a cell-containing fluid sample. The devices may be used, e.g., to detect the presence of an intracellular molecular component in a cell sample.

Analytical devices having mesoscale flow channels and cell handling chambers can be designed and fabricated in large quantities from a solid substrate material. They can be sterilized easily. Silicon is a preferred substrate material because of the well-developed technology permitting its precise and efficient fabrication, but other materials may be used, including polymers such as polytetrafluoroethylenes. The sample inlet port and other ports, the mesoscale flow system, including the sample flow channel(s) and the fluid handling region(s), and other functional elements, may be fabricated inexpensively in large quantities from a silicon substrate by any of a variety of micromachining methods known to those skilled in the art. The micromachining methods available include film deposition processes such as spin coating and chemical vapor deposition, laser fabrication or photolithographic techniques such as UV or X-ray processes, or etching methods which may be performed by either wet chemical processes or plasma processes. (See, e.g., Manz et al., Trends in Analytical Chemistry 10: 144-149 (1991)).

Flow channels of varying widths and depths can be fabricated with mesoscale dimensions. The silicon substrate containing a fabricated mesoscale flow channel may be covered and sealed with a thin anodically bonded glass cover. Other clear or opaque cover materials may be used. Alternatively, two silicon substrates can be sandwiched, or a silicon substrate can be sandwiched between two glass covers. The use of a transparent cover results in a window which facilitates dynamic viewing of the channel contents, and allows optical probing of the mesoscale flow system either visually or by machine. Other fabrication approaches may be used.

The capacity of the devices is very small, and therefore the amount of sample fluid required for an analysis is low. For example, in a 1 cm x 1 cm silicon substrate, having on its surface an array of 500 grooves which are 10 microns wide x 10 microns deep x 1 cm (10⁴ microns) long, the volume of each groove is 10⁻³ µL and the total volume of the 500 grooves is 0.5 µL. The low volume of the mesoscale flow systems allows assays to be performed on very small amounts of a liquid sample (<5 µl). The mesoscale flow systems of the devices may be microfabricated with microliter volumes or alternatively nanoliter volumes or less, which advantageously limits the amount of sample and/or reagent fluids required for the assay. In one embodiment, electron micrographs of biological structures such as circulatory networks may be used as masks for fabricating mesoscale flow systems on the substrate. Mesoscale flow systems may be fabricated in a range of sizes and conformations.

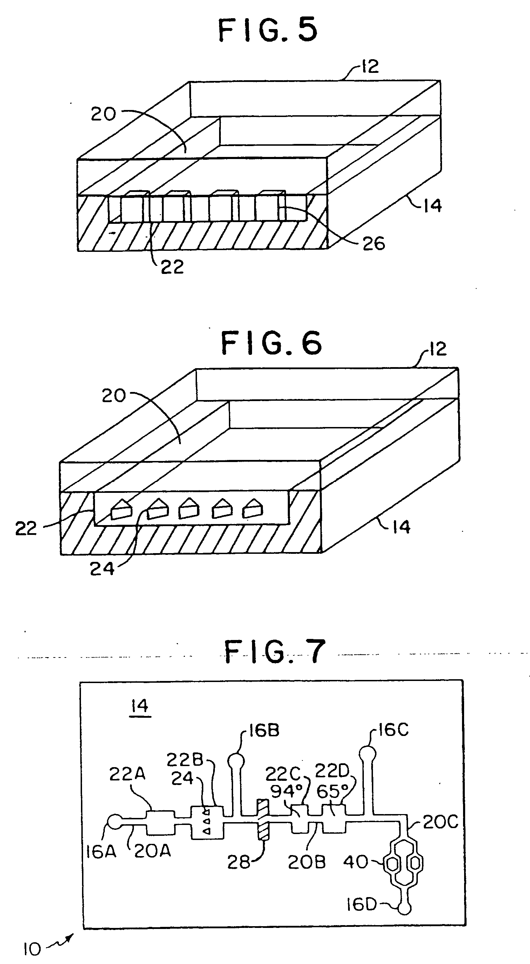

In one embodiment, the devices may be utilized to analyze a cell-containing fluid sample. The fluid handling region may comprise, in one embodiment, a cell lysing means, to allow cells in a fluid sample to be lysed prior to analysis for an intracellular molecule such as an mRNA or DNA molecule. As illustrated in Figure 6, the cell lysing means may comprise cell membrane piercing protrusions 24, extending from a surface of cell handling region 22. The device may include means, such as a pump for inducing flow through the flow system. As fluid flow is forced through the piercing protrusions 24, cells are ruptured. Cell debris may be filtered off using a filter microfabricated in the flow system downstream from the cell lysis means. The cell lysis region may also comprise sharp edged particles, e.g., fabricated from silicon, trapped within the cell handling region. In addition, the cell lysis means may comprise a region of restricted cross-sectional dimension, which implements cell lysis upon application of sufficient flow pressure. In another embodiment, the cell lysis means may comprise a cell lysing agent.

The devices may include a mesoscale detection region microfabricated in the mesoscale flow system, in fluid communication with a cell lysis region, comprising binding moieties capable of binding to a selected intracellular molecular component in the cell sample. Binding moieties may be introduced into the detection region via an inlet port in fluid communication with the detection region. Alternatively, binding moieties may be immobilized in the detection region either by physical absorption onto the channel surfaces, or by covalent attachment to the channel surfaces, or to solid phase reactant such as a polymeric bead. Techniques available in the art may be utilized for the chemical activation of silaceous surfaces, and the subsequent attachment of a binding moiety to the surfaces. (See, e.g., Haller in: Solid Phase Biochemistry, W.H. Scouten, Ed., John Wiley, New York, pp 535-597 (1983); and Mandenius et al., Anal. Biochem., 137:106-114(1984), and Anal. Biochem., 170:68-72 (1988)).

The binding moiety in the detection region may comprise, e.g., an antigen binding protein, a DNA probe, or one of a ligand/receptor pair, to enable the detection of a preselected cellular, intracellular, or other analyte, such as an antigen, a polynucleotide or a cell surface molecule. The binding assays available in the art which may be utilized in the detection region include immunoassays, enzymatic assays, ligand/binder assays and DNA hybridization assays. The detection of a particular intracellular analyte may be implemented by the selection of an appropriate binding moiety in detection region. The detection region may be fabricated according to methods disclosed in USSN 07/877,702, filed May 1, 1992 (corresponding to PCT WO 93/22053, published November 11, 1993).

The mesoscale detection region may also comprise a region sensitive to changes in flow properties induced by the presence of a preselected cellular, intracellular or other analyte in the fluid sample. The flow sensitive region may comprise, e.g., a fractal region, comprising bifurcations leading to plural secondary flow channels. The flow sensitive region, e.g., the fractal region, may be constructed in accordance with the copending related application U.S. Serial No. [Attorney Docket No. UPA002 (8261/3)], Analysis Based on Flow Restriction.

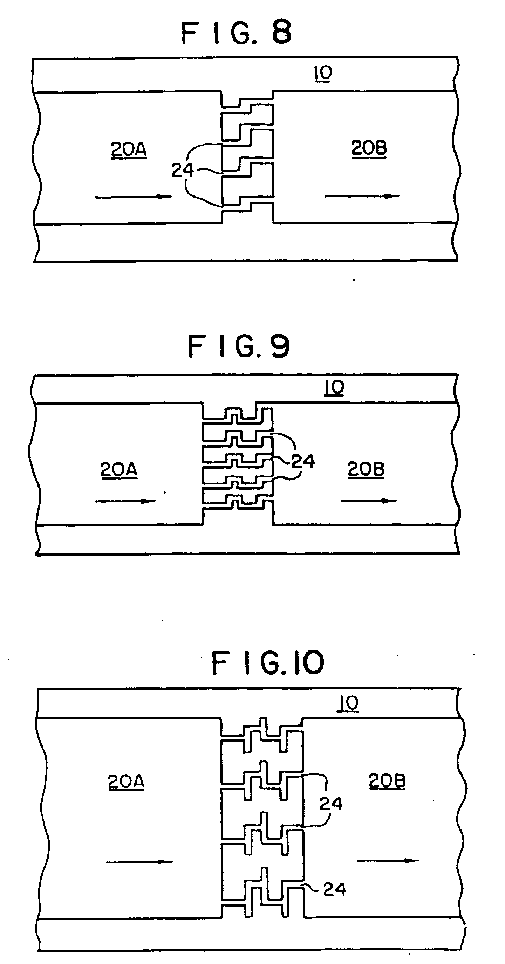

The devices may comprise a plurality of fluid handling regions to enable, e.g., the detection of a preselected intracellular or cell surface moiety in a cell-containing fluid sample. In one embodiment, the mesoscale flow system may be microfabricated with a cell lysis means, a filter for filtering cell debris, and a detection region. The filter may be micro-fabricated in the flow system between the cell lysis means and the detection region to enable the removal of the lysed cell membrane and other cell debris components from the sample, prior to detection of an intracellular analyte in the detection region. Filters which may be microfabricated in the flow system include the filters shown in Figures 8 through 10. In the device 10, shown in Figures 8 through 10, the filter 80 is microfabricated between the flow channels 20A and 20B allowing sample fluid in channel 20A to pass through the filter. The filtrate exits through the filter into channel 20B, prior to subsequent downstream analysis in, e.g., a mesoscale detection region. The filter is a mesoscale flow channel of reduced diameter in comparison with channel 20, microfabricated with depths and widths on the order of 0.1 to 20 µm. In contrast, the flow channels 20A and 20B have increased widths and depths on the order of a maximum of approximately 500 µm. The smaller diameter of filter 80 allows the filtration of sheared cell membranes and other cell debris from the sample. Other filter means may be utilized, such as the posts 26 extending from a wall of the flow channel 20 shown in Figure 5.

The presence of an analyte in the detection region can be detected by any of a number of methods including monitoring the pressure or electrical conductivity of sample fluids in selected regions of the flow system in the device, or by optical detection through a transparent cover or a translucent section of the substrate itself, either visually or by machine. The detection of an analyte in the detection region may be implemented as is disclosed in the copending related applications USSN 07/877,702, filed May 1, 1992 (corresponding to PCT WO 93/22053, published November 11, 1993), and USSN 07/877,701, filed May 1, 1992 (corresponding to PCT WO 93/22054, published November 11, 1993). Devices such as valves, mesoscale pressure sensors, and other mechanical sensors can be fabricated directly on the silicon substrate and can be mass-produced according to established technologies. Angell et al., Scientific American, 248:44-55 (1983). Pressure sensors and other detection means also may be provided in an appliance utilized in combination with the device.

In another embodiment, the fluid handling region may comprise a cell capture region for separating a preselected cell population from a cell-containing fluid sample, to enable the downstream analysis of a macromolecule on or within the cells, or of a component in the extracellular fluid. The cell capture region may comprise binding moieties capable of reversibly binding a target cell via a characteristic cell surface molecule such as protein. In one embodiment, the cell capture region may be utilized to isolate a preselected cell population from a cell containing fluid sample. In this embodiment, the device is provided with means for inducing flow of the sample through the flow system, such as a pump. At a low flow pressure, the cells bind to the binding moieties in the cell capture region. Flow is then continued to wash the cells, e.g., with a flow of buffer. At higher flow rates and pressures, the washed cells are released from the separation region and move downstream for analysis in, e.g., a mesoscale detection region. In another embodiment, the cells remain immobilized while extracellular fluid fluid flows downstream and is analyzed in, e.g., a mesoscale detection region. The bound cells may also be removed from the cell capture region by flowing a specific solvent through the flow system, capable of desorbing the cells from the wall of the cell capture region.

The binding moiety, capable of binding the cells in the cell capture region, e.g., via a cell surface molecule, may be immobilized on the surface of the mesoscale flow channels by physical absorption onto the channel surfaces, or by chemical activation of the surface and subsequent attachment of biomolecules to the activated surface. Techniques available in the art may be utilized for the chemical activation of silaceous channel surfaces, and for the subsequent attachment of a binding moiety to the surfaces. (See, e.g., Haller in: Solid Phase Biochemistry, W.H. Scouten, Ed., John Wiley, New York, pp. 535-597 (1983); and Mandenius et al., Anal. Biochem., 137:106-114 (1984), and Anal. Biochem., 170:68-72 (1988)). The binding moiety may be provided within the cell capture region of the mesoscale flow system, as disclosed in USSN 07/877,702, filed May 1, 1992 (corresponding to PCT WO 93/22053, published November 11, 1993). The capture of a particular cell type can be implemented by selecting the appropriate binding moiety.

As illustrated in Figure 5, the cell handling region 22 may comprise protrusions 26 constituting a cellular sieve for separating cells by size. As cell samples are flowed, typically under low pressure, through the flow channel, only cells capable of passing between the protrusions 26 are permitted to flow through in the flow channel.

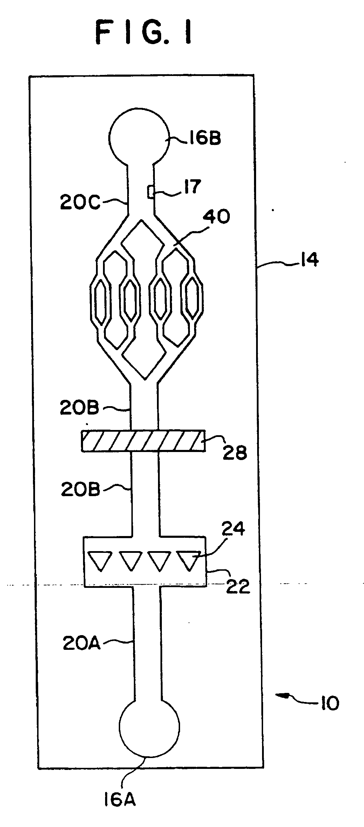

The devices may comprise several different cell handling regions in the mesoscale flow system of one device. In one embodiment, illustrated schematically in Figures 1, 2 and 3, the device 10 may include a silicon substrate 14 micro-fabricated with a mesoscale flow channel 20, cell lysis chamber 22, and the fractal detection region 40. The device may be utilized to detect the presence of a preselected intracellular component of a cell sample. The cell lysis chamber 22 is provided with cell membrane piercing protrusions 24. Sample fluid may be added to the flow system through inlet 16A. A pump in the device then may be used to force a cell sample through flow channel 20A to the cell lysis chamber 22. The lysed cell sample is then filtered through filter 28 and flows through the fractal detection region 40 towards port 16B. The substrate 14 is covered with a glass or plastic window 12. The presence of an intracellular analyte is indicated by the detection, e.g., optically, of flow restriction in the fractal detection region 40, induced by the particular intracellular analyte. The fractal region may include binding moieties, capable of binding to the analyte, to enhance flow restriction in the fractal region 40.

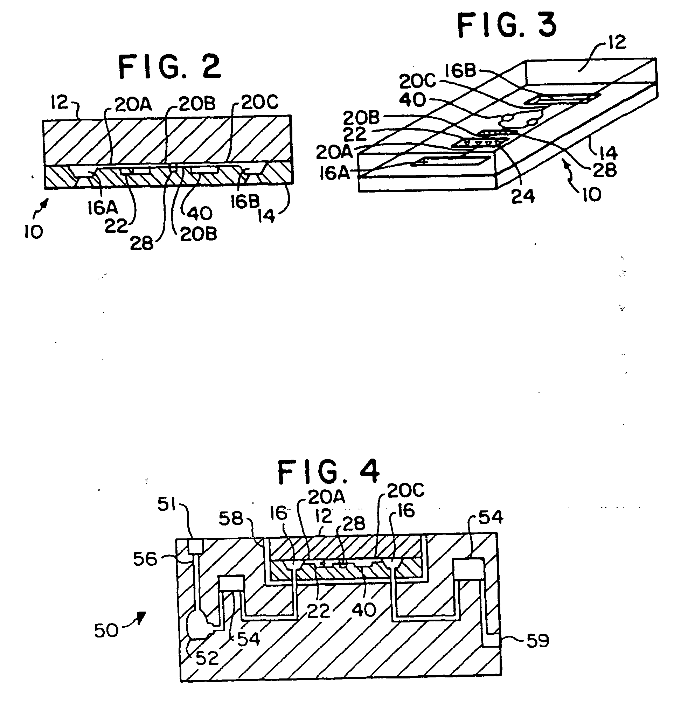

The analytical devices containing the mesoscale flow system can be used in combination with an appliance for delivering and receiving fluids to and from the devices, such as appliance 50, shown schematically in Figure 4, which incorporates a nesting site 58 for holding the device 10, and for registering ports, e.g., ports 16 on the device 10, with a flow line 56 in the appliance. The appliance may include means, such as a pump, for forcing the cell containing sample into a cell lysis means to cause cell lysis upon application of sufficient flow pressure. After a cell containing fluid sample suspected to contain a particular cellular analyte is applied to the inlet port 51 of the appliance, pump 52 is actuated to force the sample through the flow system 20 of device 10. Alternatively, depending on the analytical device in use, the sample may be injected into the device, or may enter the flow system simply by capillary action. In one embodiment, the flow systems of the devices may be filled to a hydraulically full volume and the appliance may be utilized to direct fluid flow through the flow system.

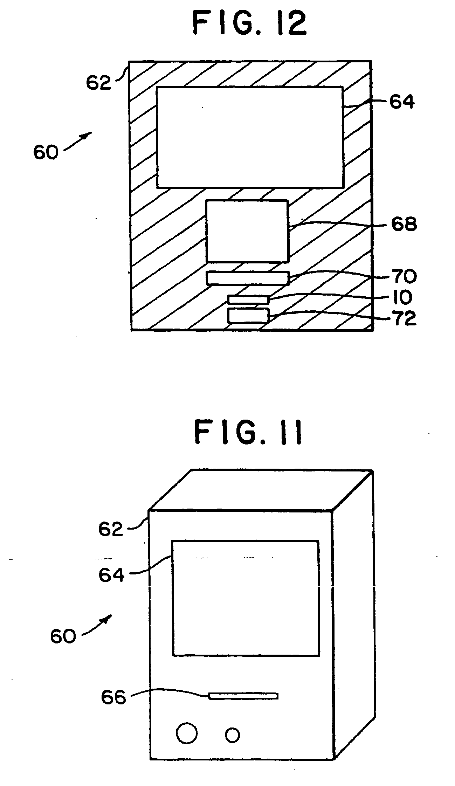

The analytical devices also may be utilized in combination with an appliance for viewing the contents of the mesoscale channels in the devices. The appliance in one embodiment may comprise a microscope for viewing the contents of the mesoscale channels in the devices. In another embodiment, a camera may be included in the appliance, as illustrated in the appliance 60 shown schematically in Figures 11 and 12. The appliance 60 is provided with a housing 62, a viewing screen 64 and a slot 66 for inserting a chip into the appliance. As shown in cross section in Figure 12, the appliance 60 also includes a video camera 68, an optical system 70, and a tilt mechanism 72, for holding device 10, and allowing the placement and angle of device 10 to be adjusted manually. The optical system 70 may include a lens system for magnifying the channel contents as well as a light source. The video camera 68 and screen 64 allow analyte induced changes in sample fluid properties, such as flow properties or color, to be monitored visually, and optionally recorded using the appliance.

The devices of the invention may be utilized to implement a variety of automated, sensitive and rapid analyses of a fluid sample. The device may be fabricated with a series of fluid handling regions in one flow system to enable the rapid efficient multistep analysis of a fluid cell containing sample on a microvolume scale. The devices may also include two or more separated flow systems, e.g., with a common inlet port, wherein one flow system is adapted as a control, such that data obtained during an analysis can be compared with data from the control flow system. A range of analyses thus may be implemented in one device.

In one embodiment, the device of the invention may comprise three or more inlet ports and a branching flow channel in fluid communication with the ports. The device may be provided with valves, e.g., in the appliance, for opening and closing the ports, to control the flow of fluid through the flow system. As illustrated in the device 10, shown schematically in Figure 7, ports 16A, 16B, 16C and 16D may be independently opened or closed, by means of valves in, e.g., an appliance used in combination with the device, to allowfluid in the flow system to be directed, e.g., out via port 16 or, alternatively, to the fractal detection region 40 and port 16D.

The invention will be understood further from the following nonlimiting examples.

### Example 1

A channel containing a barrier 26 with 7µm gaps (illustrated in cross section in Figure 5) is filled with HTF-BSA medium and a semen sample applied at the entry hole. The progression of the sperm through the barrier serves as an indicator of sperm motility, and is compared with a control sample.

### Example 2

Figure 7 depicts schematically a device 10 including substrate 14 used to separate and detect a nucleic acid from a subpopulation of cells in a mixture in a biological fluid sample. Microfabricated on device 10 is a mesoscale flow path 20 which includes a cell separation chamber 22A, a cell lysis chamber 22B, a filter region 28, a polymerase chain reaction (PCR) chamber comprising sections 22C and 22D, and a fractal detection region 40. The mesoscale flow system 20 is also provided with fluid entry/exit ports 16A, 16B, 16C and 16D. The device is used in combination with an appliance, such as appliance 50, shown in Figure 4. The appliance is provided with fluid paths mated to ports 16 in the device, and valves allowing the ports 16 to be mechanically closed and opened. The appliance also includes pump 52 for regulating the flow of sample fluid through the device. The appliance further includes means for heating the PCR reaction chamber sections 22C and 22D in the device.

Initially, valves in the appliance are used to close ports 16C and 16D, while ports 16A and 16B are open. A sample containing a mixture of cells is directed to the sample inlet port 16A by the pump 52 in the appliance, and flows through the mesoscale flow path 20 to separation chamber 22A. Chamber 22A contains binding moieties immobilized on the wall of the chamber which selectively bind to a surface molecule on a desired type of cell in the sample. Remaining cellular components exit the substrate via port 16B. After binding of the desired cell population in chamber 22A, flow with buffer is continued, to wash and assure isolation of the cell population. Next port 16B is closed and 16C is opened. Flow is then increased sufficiently to dislodge the immobilized cells. Flow is continued, forcing cells through membrane piercing protrusions 24 in chamber 22B, which tear open the cells releasing intracellular material.

Sample flow continues past filter 28, which filters off large cellular membrane components and other debris, to mesoscale PCR chamber section 22C, which is connected to PCR chamber section 22D by flow channel 20B. Taq polymerase, primers and other reagents required for the PCR assay next are added to section 22D through port 16C from a mated port and flow path in the appliance, permitting mixing of the intracellular soluble components from the separated subpopulation of cells and the PCR reagents. With port 16A closed, a pump in the appliance connected via port 16B is used to cycle the PCR sample and reagents through flow channel 20B between sections 22C and 22D, set at 94°C and 65°C respectively, to implement plural polynucleotide melting and polymerization cycles, allowing the amplification of product polynucleotide. The mesoscale PCR analysis is performed in accordance with methods disclosed in USSN 07/877,662, filed May 1, 1992 (corresponding to PCT WO 93/22058, published November 11, 1993).

The valves in the appliance next are used to close port 16C and to open port 16D. The pump in the appliance connected to port 16B is then used to direct the amplified polynucleotide isolated from the cell population to the fractal detection region 40. Flow restriction in the fractal region 40 serves as a positive indicator of the presence of amplified polynucleotide product and is detected optically through a glass cover disposed over the detection region.

## Claims

1. A device for analyzing a fluid, cell-containing sample, the device comprising:
a solid substrate microfabricated to define;
a sample inlet port; and
a mesoscale flow system defined by chambers and flow passages having cross-sectional dimensions of about 0.1-500 µm wherein chambers in the substrate can also have larger dimensions of a few millimetres, the mesoscale flow system comprising:
a sample flow channel extending from said inlet port; and
a cell handling region for treating cells disposed in fluid communication with said flow channel, said cell handling region comprising a cell lysing structure;
means for inducing flow of cells in said sample through said mesoscale flow channel and said cell handling region to force cells in said sample into contact with said cell lysing structure, thereby to lyse cells in said sample; and
means downstream of said cell lysing structure for detecting an intracellular molecular component of a cell in said lysed cell sample.

2. The device according to claim 1 wherein said cell lysing structure comprises:
(a) a portion of the flow channel having cell membrane piercing protrusions extending from the wall thereof; or,
(b) sharp edged particles trapped within said cell handling region; or,
(c) a region of restricted cross-sectional dimension sufficient to permit the passage of intracellular molecules while prohibiting the passage of cells.

3. The device of claim 1 or claim 2 further comprising means disposed downstream of said cell lysing structure for collecting insoluble cellular debris.

4. The device of claim 1 or claim 2 further comprising a filter means disposed downstream of said cell lysing structure.

5. The device of claim 1 or claim 2 wherein said cell handling region further comprises a cell capture region comprising immobilized binding sites which reversibly bind a preselected cell surface molecule of a cell population in a cell-containing fluid sample; and
wherein said means for inducing flow comprises means for inducing flow of said cell-containing sample:
at a first flow rate sufficiently slow to permit capture of cells in said cell population by said binding sites, thereby to separate said cell population from said sample; and
at a second flow rate, higher than said first flow rate, and sufficient to release said separated cells from said capture region, and to deliver said separated cells to said cell lysing structure.

6. The device of claim 5 wherein said detecting means comprises means downstream of said cell capture region for determining the presence of an extracellular component of said sample.

7. The device of claim 5 wherein said detecting means comprises means for detecting the presence of an intracellular component in said captured cells.

8. The device of claim 7 further comprising filter means, disposed between said cell-lysing means and said detection means, for filtering cellular debris from said sample.

9. The device of claim 8 further comprising a sump for collecting insoluble debris disposed adjacent said filter.

10. The device of claim 1 or claim 2 wherein said cell handling structure further comprises a cell sieve comprising means defining a plurality of flow passages of restricted size allowing only cells of a sufficiently small diameter to pass there through.

11. The device of any one of the preceding claims wherein said substrate comprises microfabricated silicon.

12. The device of any one of claims 1, 2 or 11 further comprising an appliance for use in combination with said substrate, said appliance comprising:
means for holding said substrate; and
fluid input means interfitting with an inlet port on said substrate; and
wherein said means for inducing flow comprises pump means, disposed in said appliance, for passing fluid through the flow system of said substrate when it is held in said holding means.

13. The device of any one of claims 1, 2 or 11 wherein said means for detecting comprises an appliance for use in combination with said substrate, said appliance comprising:
means for holding said substrate; and
optical means for viewing the contents of said mesoscale flow system in said substrate.

14. The device of claim 13, wherein said optical means comprises magnifying optics and a video camera, and wherein said appliance further comprises:
a tilt mechanism for manually adjusting the angle and location of the device; and
a video screen for viewing the contents of said flow system.

15. The device of any one of claims 1, 2 or 11 wherein said mesoscale flow system further comprises:
a branching channel in fluid communication with said flow channel; and
at least two additional ports communicating between said flow channel and said branching channel, respectively, and the exterior of said flow system; and
wherein said device further comprises valve means for directing flow through said flow system to a selected one of said additional ports.

16. The device of claim 15 further comprising an appliance for use in combination with said substrate, said appliance comprising:
means for holding said substrate;
fluid flow channels interfitting with at least two of said ports when said substrate is held in said holding means; and
wherein said means for inducing flow comprises pump means disposed within said appliance in fluid communication with said inlet ports for inducing flow within said flow system.

17. The device of claim 16 wherein said valve means is disposed within said appliance.

18. The device of any one of claims 1, 2 or 11 wherein said detecting means comprises:
at least two analyte detection regions in said flow system, in fluid communication with said cell handling structure, one of said detection regions being adapted to analyze a sample, the other being adapted as a control; and
wherein said means for inducing flow comprises means for inducing flow of a sample through said cell handling structure and then to both said detection regions, thereby to permit comparison of data from the detection regions.

19. The device of any one of claims 1, 2, 11 or 15 wherein said detecting means comprises a detection region within said mesoscale flow system for optically or electrically gathering data indicative of the presence or concentration of an analyte in a sample contained within said flow system.

20. A device for analyzing a fluid, cell-containing sample, the device comprising:
a solid substrate microfabricated to define:
a sample inlet port; and
a mesoscale flow system defined by chambers and flow passages having cross-sectional dimensions of about 0.1-500 µm wherein chambers in the substrate can also have larger dimensions of a few millimetres, the mesoscale flow system comprising:
a sample flow channel extending from said inlet port; and
a cell handling region for treating cells disposed in fluid communication with said flow channel, said cell handling region comprising a cell lysing agent;
means for inducing flow of cells in a sample through said mesoscale flow channel and said cell handling region to force cells in said sample into contact with said cell lysing agent, thereby to lyse cells in said sample; and
means downstream of said cell handling region for detecting an intracellular molecular component of a cell in said lysed cell sample.

## Patentansprüche

1. Vorrichtung zum Analysieren einer flüssigen, zellenhältigen Probe, wobei die Vorrichtung Folgendes umfasst:
ein festes Substrat, das mikrobearbeitet ist; um Folgendes zu definieren:
eine Probeneinlassöffnung; und
ein mesoskaliges Durchflusssystem, das durch Kammern und Durchflussdurchgänge mit Querschnittsabmessungen von etwa 0,1 bis 500 µm definiert ist, worin Kammern im Substrat auch größere Abmessungen von einigen Millimetern haben können, wobei das mesoskalige Durchflusssystem Folgendes umfasst:
einen Probendurchflusskanal, der sich von der Einlassöffnung weg erstreckt; und
einen Zellenbehandlungsbereich zur Behandlung von Zellen, der in Fluidkommunikation mit dem Durchflusskanal angeordnet ist, wobei der Zellenbehandlungsbereich eine Zelllysestruktur umfasst;
ein Mittel zum Induzieren eines Zellenflusses in einer Probe durch den mesoskaligen Durchflusskanal und den Zellenbehandlungsbereich, um Zellen in der Probe in Kontakt mit der Zelllysestruktur zu drängen und **dadurch** Zellen in der Probe zu lysieren; und
ein stromabwärts von der Zelllysestruktur angeordnetes Mittel zur Detektion einer intrazellulären, molekularen Komponente einer Zelle in der lysierten Zellprobe.

2. Vorrichtung nach Anspruch 1, worin die Zelllysestruktur Folgendes umfasst:
(a) einen Abschnitt des Durchflusskanals mit Zellmembranen durchstoßenden Vorsprüngen, die sich von seiner Wand weg erstrecken; oder
(b) scharfkantige Teilchen, die innerhalb des Zellbehandlungsbereichs eingeschlossen sind; oder
(c) einen Bereich mit eingeschränktem Querschnitt, der ausreicht, um das Hindurchtreten intrazellulärer Moleküle zu ermöglichen, während das Hindurchtreten von Zellen verhindert wird.

3. Vorrichtung nach Anspruch 1 oder 2, die weiters stromabwärts von der Zelllysestruktur angeordnete Mittel zum Sammeln unlöslicher Zellbruchstücke umfasst.

4. Vorrichtung nach Anspruch 1 oder 2, die weiters ein stromabwärts von der Zelllysestruktur angeordnetes Filtermittel umfasst.

5. Vorrichtung nach Anspruch 1 oder 2, worin der Zellenbehandlungsbereich weiters einen Zelleinfangbereich umfasst, der immobilisierte Bindungsstellen umfasst, die ein vorgewähltes Zelloberflächenmolekül einer Zellpopulation in einer zellenhältigen, flüssigen Probe reversibel binden; und
worin das Flussinduktionsmittel Mittel umfasst, um den Fluss der zellenhältigen Probe
mit einer ersten Flussrate, die ausreichend langsam ist, um das Einfangen von Zellen in der Zellpopulation durch die Bindungsstellen zu ermöglichen, wodurch die Zellpopulation von der Probe abgetrennt wird; und
mit einer zweiten Flussrate, die höher ist als die erste Flussrate und ausreicht, um die abgetrennten Zellen aus dem Einfangbereich freizusetzen und die abgetrennten Zellen an die Zelllysestruktur weiterzuleiten; herbeizuführen.

6. Vorrichtung nach Anspruch 5, worin das Detektionsmittel stromabwärts vom Zelleinfangbereich befindliche Mittel zum Nachweis der Gegenwart einer extrazellulären Komponente der Probe umfasst.

7. Vorrichtung nach Anspruch 5, worin das Detektionsmittel Mittel zum Nachweis der Gegenwart einer intrazellulären Komponente in den eingefangenen Zellen umfasst.

8. Vorrichtung nach Anspruch 7, die weiters zwischen dem Zelllysemittel und dem Detektionsmittel angeordnete Filtermittel zum Abfiltrieren von Zellbruchstücken aus der Probe umfasst.

9. Vorrichtung nach Anspruch 8, die weiters einen angrenzend an den Filter angeordneten Sumpf zum Sammeln unlöslicher Bruchstücke umfasst.

10. Vorrichtung nach Anspruch 1 oder 2, worin die Zellbehandlungsstruktur weiters ein Zellensieb umfasst, das Mittel umfasst, die eine Vielzahl an Durchflussdurchgängen mit eingeschränkter Größe definieren, welche nur Zellen mit einem ausreichend kleinen Durchmesser hindurchlassen.

11. Vorrichtung nach einem der vorangegangenen Ansprüche, worin das Substrat mikrobearbeitetes Silicium umfasst.

12. Vorrichtung nach einem der Ansprüche 1, 2 oder 11, die weiters eine Gerätschaft zur Verwendung in Kombination mit dem Substrat umfasst, wobei die Gerätschaft Folgendes umfasst:
ein Mittel zum Halten des Substrats; und
ein Flüssigkeitszufuhrmittel, das an eine Einlassöffnung des Substrats angepasst ist; und
worin das Flussinduktionsmittel in der Gerätschaft angeordnete Pumpmittel zum Hindurchschicken von Flüssigkeit durch das Durchflusssystem des Substrats umfasst, wenn dieses im Haltemittel gehalten wird.

13. Vorrichtung nach einem der Ansprüche 1, 2 oder 11, worin das Detektionsmittel eine Gerätschaft zur Verwendung in Kombination mit dem Substrat umfasst, wobei die Gerätschaft Folgendes umfasst:
ein Mittel zum Halten des Substrats; und
ein optisches Mittel zum Betrachten des Inhalts des mesoskaligen Durchflusssystems im Substrat.

14. Vorrichtung nach Anspruch 13, worin das optische Mittel eine Vergrößerungsoptik und eine Videokamera umfasst und worin die Gerätschaft weiters umfasst:
einen Kippmechanismus zum manuellen Einstellen des Winkels und der Position der Vorrichtung; und
einen Videobildschirm zum Betrachten des Inhalts des Durchflusssystems.

15. Vorrichtung nach einem der Ansprüche 1, 2 oder 11, worin das mesoskalige Durchflusssystem weiters Folgendes umfasst:
eine in Fluidkommunikation mit dem Durchflusskanal stehende Kanalverzweigung; und
zumindest zwei zusätzliche Öffnungen, die mit dem Durchflusskanal bzw. der Kanalverzweigung und dem Außenraum des Durchflusssystems kommunizieren; und
worin die Vorrichtung weiters Ventilmittel umfasst, um den Fluss durch das Durchflusssystem hindurch zu einer gewählten zusätzlichen Öffnung hin zu führen.

16. Vorrichtung nach Anspruch 15, die weiters eine Gerätschaft zur Verwendung in Kombination mit dem Substrat umfasst, wobei die Gerätschaft Folgendes umfasst:
ein Mittel zum Halten des Substrats;
Fluidflusskanäle, die an zumindest zwei der Öffnungen angepasst sind, wenn das Substrat im Haltemittel gehalten wird; und
worin das Flussinduktionsmittel ein Pumpmittel umfasst, das innerhalb der Gerätschaft in Fluidkommunikation mit den Einlassöffnungen angeordnet ist, um innerhalb des Durchflusssystems eine Strömung zu induzieren.

17. Vorrichtung nach Anspruch 16, worin das Ventilmittel innerhalb der Gerätschaft angeordnet ist.

18. Vorrichtung nach einem der Ansprüche 1, 2 oder 11, worin das Detektionsmittel Folgendes umfasst:
zumindest zwei Analyten-Detektionsbereiche im Durchflusssystem in Fluidkommunikation mit der Zellenbehandlungsstruktur, wobei einer der Detektionsbereiche dazu ausgebildet ist, eine Probe zu analysieren, und der andere als Vergleich vorgesehen ist; und
worin das Flussinduktionsmittel Mittel zum Induzieren des Flusses einer Probe durch die Zellenbehandlungsstruktur hindurch und dann zu den beiden Detektionsbereichen hin umfasst, wodurch ein Vergleich der Daten aus den Detektionsbereichen möglich wird.

19. Vorrichtung nach einem der Ansprüche 1, 2, 11 oder 15, worin das Detektionsmittel einen Detektionsbereich innerhalb des mesoskaligen Durchflusssystems zur optischen oder elektrischen Datenerfassung umfasst, die Aufschluss über die Gegenwart oder die Konzentration eines Analyten in einer innerhalb des Durchflusssystems enthaltenen Probe geben.

20. Vorrichtung zum Analysieren einer flüssigen, zellenhältigen Probe, wobei die Vorrichtung Folgendes umfasst:
ein festes Substrat, das mikrobearbeitet ist, um Folgendes zu definieren:
eine Probeneinlassöffnung; und
ein mesoskaliges Durchflusssystem, das durch Kammern und Durchflussdurchgänge mit Querschnittsabmessungen von etwa 0,1 bis 500 µm definiert ist, worin Kammern im Substrat auch größere Abmessungen von einigen Millimetern haben können, wobei das mesoskalige Durchflusssystem Folgendes umfasst:
einen Probendurchflusskanal, der sich von der Einlassöffnung weg erstreckt; und
einen Zellenbehandlungsbereich zur Behandlung von Zellen, der in Fluidkommunikation mit dem Durchflusskanal angeordnet ist, wobei der Zellenbehandlungsbereich ein Zelllysierungsmittel umfasst;
ein Mittel zum Induzieren des Flusses von Zellen in einer Probe durch den mesoskaligen Durchflusskanal und den Zellenbehandlungsbereich, um Zellen in der Probe in Kontakt mit dem Zelllysierungsmittel zu drängen und **dadurch** Zellen in der Probe zu lysieren; und
ein stromabwärts vom Zellenbehandlungsbereich angeordnetes Mittel zur Detektion einer intrazellulären, molekularen Komponente einer Zelle in der lysierten Zellprobe.

## Revendications

1. Dispositif pour analyser un échantillon contenant des cellules, fluide, le dispositif comprenant:
un substrat solide microfabriqué pour définir:
un orifice d'entrée d'échantillon, et
un système d'écoulement à échelle intermédiaire défini par des chambres et des passages d'écoulement ayant des dimensions en section transversale d'environ 0,1 à 500 µm où les chambres dans le substrat peuvent également avoir des dimensions plus grandes de quelques millimètres, le système d'écoulement à échelle intermédiaire comprenant:
un canal d'écoulement d'échantillon s'étendant à partir dudit orifice d'entrée;
une région de manipulation des cellules pour traiter les cellules disposées en communication de fluide avec ledit canal d'écoulement, ladite région de manipulation des cellules comprenant une structure de lyse des cellules;
un moyen pour induire l'écoulement des cellules dans l'échantillon à travers ledit canal d'écoulement à échelle intermédiaire et ladite région de manipulation des cellules pour forcer les cellules dans ledit échantillon en contact avec ladite structure de lyse des cellules, pour ainsi lyser les cellules dans ledit échantillon; et
un moyen en aval de ladite structure de lyse de cellules pour détecter un composant intracellulaire moléculaire d'une cellule dans ledit échantillon de cellules lysées.

2. Dispositif selon la revendication 1 dans lequel ladite structure de lyse des cellules comprend:
(a) une portion du canal d'écoulement ayant des protubérances perçant la membrane des cellules s'étendant à partir de la paroi de celui-ci; ou
(b) des particules à bords aigus piégées dans ladite région de maintien des cellules; ou,
(c) une région d'une dimension en section transversale restreinte suffis ante pour permettre le passage des molécules intracellulaires tout en empêchant le passage des cellules.

3. Dispositif de la revendication 1 ou la revendication 2 comprenant de plus un moyen disposé en aval de ladite structure de lyse de cellules pour col lecter les débris cellulaires insolubles.

4. Dispositif de la revendication 1 ou la revendication 2 comprenant de plus un moyen de filtre en aval de ladite structure de lyse des cellules.

5. Dispositif de la revendication 1 ou la revendication 2 dans lequel ladite région de manipulation des cellules comprend de plus une région de capture des cellules comprenant des sites de liaison immobilisés qui lient réversiblement une molécule de surface cellulaire présélectionnée d'une population de cellules dans un échantillon de fluide contenant des cellules; et
dans lequel ledit moyen pour induire l'écoulement comprend un moyen pour induire un écoulement dudit échantillon contenant des cellules:
à une première vitesse d'écoulement suffisamment lente pour permet tre la capture des cellules dans ladite population de cellules par des sites de liaison, pour ainsi séparer ladite population de cellules dudit échantillon; et
à une seconde vitesse d'écoulement, plus élevée que ladite première vitesse d'écoulement, et suffisante pour libérer lesdites cellules de ladite région de capture, et pour amener lesdites cellules séparées à ladite structure de lyse des cellules.

6. Dispositif de la revendication 5 dans lequel ledit moyen de détection comprend un moyen en aval de ladite région de capture des cellules pour déterminer la présence d'un composant extracellulaire dudit échantillon.

7. Dispositif de la revendication 5 dans lequel ledit moyen de détection comprend un moyen pour détecter la présence d'un composant intracellulaire dans lesdites cellules capturées.

8. Dispositif de la revendication 7 comprenant un moyen de filtre, disposé entre ledit moyen de lyse des cellules et ledit moyen de détection pour filtrer les débris cellulaires provenant dudit échantillon.

9. Dispositif de la revendication 8 comprenant de plus un récipient d'évacuation des déchets pour collecter les débris insolubles disposé adjacent audit filtre.

10. Dispositif de la revendication 1 ou la revendication 2 dans lequel ladite structure de manipulation des cellules comprend de plus un tamis cellulaire comprenant un moyen définissant une pluralité de passages d'écoulement de taille restreinte permettant à seulement des cellules d'un diamètre suffisamment petit de traverser.

11. Dispositif selon l'une quelconque des revendications précédentes dans lequel ledit substrat comprend du silicium microfabriqué.

12. Dispositif selon l'une quelconque des revendications 1, 2 ou 11 comprenant de plus un appareillage pour utilisation en combinaison avec ledit substrat, ledit appareillage comprenant:
un moyen pour maintenir ledit substrat; et
un moyen d'entrée de fluide s'interadaptant avec un orifice d'entrée sur ledit substrat; et
dans lequel ledit moyen pour induire l'écoulement comprend un moyen de pompage, disposé dans ledit appareillage, pour passer le fluide à travers le système d'écoulement dudit substrat lorsqu'il est maintenu dans ledit moyen de maintien.

13. Dispositif selon l'une quelconque des revendications 1, 2 ou 11 dans lequel ledit moyen de détection comprend un appareillage pour utilisation en combinaison avec ledit substrat, ledit apppareillage comprenant:
un moyen pour maintenir ledit substrat; et
un moyen optique pour visualiser les contenus du système d'écoulement à échelle intermédiaire dans ledit substrat.

14. Dispositif selon la revendication 13 , dans lequel ledit moyen optique comprend des optiques d'agrandissement et une caméra vidéo, et dans lequel ledit appareillage comprend de plus:
un mécanisme à inclinaison pour ajuster manuellement l'angle et la position du dispositif; et
un écran vidéo pour visualiser les contenus dudit système d'écoulement.

15. Dispositif selon l'une quelconque des revendications 1, 2 ou 11 dans lequel ledit système d'écoulement à échelle intermédiaire comprend de plus:
un canal de dérivation en communication de fluide avec ledit canal d'écoulement; et
au moins deux orifices additionnels communiquant entre ledit canal d'écoulement et ledit canal de dérivation, respectivement, et l'extérieur dudit système d'écoulement; et
dans lequel ledit dispositif comprend de plus un moyen de vannes pour diriger l'écoulement à travers ledit système d'écoulement vers l'un desdits orifices additionnels sélectionné.

16. Dispositif selon la revendication 15 comprenant d e plus un appareillage pour utilisation en combinaison avec ledit substrat, ledit appareillage comprenant:
un moyen pour maintenir ledit substrat;
des canaux d'écoulement de fluide s'interadaptant avec au moins deux desdits orifices lorsque ledit substrat est maintenu dans ledit moyen de maintien; et
dans lequel ledit moyen pour induire l'écoulement comprend un moyen de pompage disposé dans ledit appareillage en communication de fluide avec lesdits orifices d'entrée pour induire un écoulement dans ledit système d'écoulement.

17. Dispositif selon la revendication 16 dans lequel ledit moyen de vanne est disposé dans ledit appareillage.

18. Dispositif selon l'une quelconque des revendications 1, 2 ou 11 dans lequel ledit moyen de détection comprend:
au moins deux régions de détection d'analyte dans ledit système d'écoulement, en communication de fluide avec ladite structure de manipulation des cellules, une des régions de détection étant adaptée pour analyser un échantillon, l'autre étant adaptée en tant que contrôle; et
dans lequel ledit moyen pour induire l'écoulement comprend un moyen pour induire un écoulement d'un échantillon à travers ladite structure de manipulation des cellules et ensuite vers les deux dites régions de détection, pour permettre ain si la comparaison des données à partir des régions de détection.

19. Dispositif selon l'une quelconque des revendications 1, 2, 11 ou 15 dans lequel ledit moyen de détection comprend une région de détection dans ledit système d'écoulement à échelle intermédiaire pour collecter optiquement ou électriquement des données indicatrices de la présence ou de la concentration d'un analyte dans un échantillon contenu dans ledit système d'écoulement.

20. Dispositif pour analyser un échantillon contenant des cellules, fluide, le dispositif comprenant:
un substrat solide microfabriqué pour définir:
un orifice d'entrée d'échantillon; et
un système d'écoulement à échelle intermédiaire défini par des chambres et des passages d'écoulement ayant des dimensions en section transversale d'environ 0,1 à 500 µm où les chambres dans le substrat peuvent également avoir des dimensions plus grandes de quelques millimètres, le système d'écoulement à échelle intermédiaire comprenant:
un canal d'écoulement d'échantillon s'étendant à partir dudit orifice d'entrée; et
une région de manipulation des cellules pour traiter des cellules disposées en communication de fluide avec ledit canal d'écoulement, ladite région de manipulation des cellules comprenant un agent de lyse des cellules;
un moyen pour induire un écoulement des cellules dans un échantillon par ledit canal d'écoulement à échelle intermédiaire et ladite région de manipulation des cellules pour forcer les cellules dans ledit échantillon en contact avec ledit agent de lyse des cellules, pour ainsi lyser les cellules dans ledit échantillon; et
un moyen en aval de ladite région de manipulation des cellules pour détecter un analyte dans ledit échantillon de cellules lysées.
